# EUROPÄISCHE PATENTSCHRIFT

(11) **EP 1 169 398 B1**
(45) Veröffentlichungstag und Bekanntmachung des Hinweises auf die Patenterteilung: **23.04.2008**
(21) Anmeldenummer: 00909353.5
(22) Anmeldetag: 10.03.2000
(51) Int. Cl.: C09D 13/00, A61K 8/02

(54) **VERWENDUNG EINER ALKYLCELLULOSE UND/ODER HYDROXYALKYLCELLULOSE ZUR ERHÖHUNG DER ZUG-, BRUCH- UND BIEGEFESTIGKEIT VON FARBMINEN SOWIE SOLCHE MINEN ENTHALTENDE FARBSTIFTE**
USE OF AN ALKYLCELLULOSE AND/OR HYDROXYALKYLCELLULOSE FOR INCREASING THE TENSILE, BREAKING AND FLEXURAL STRENGTH OF COLOURED LEADS AND COLOURED PENCILS CONTAINING THE SAME
UTILISATION D'ALKYLCELLULOSE ET D'HYDROXYALKYLCELLULOSE POUR RENFORCER LA RESISTANCE A LA TRACTION, A LA RUPTURE ET A LA FLEXION DE MINES DE COULEUR ET CRAYONS DE COULEUR CONTENANT DE TELLES MINES

(30) Priorität: 16.03.1999 DE 19911748
(43) Veröffentlichungstag der Anmeldung: 09.01.2002
(73) Patentinhaber: Schwan-STABILO Cosmetics GmbH & Co. KG, 90562 Heroldsberg (DE)
(72) Erfinder: BRÜCHERT, Werner, D-90522 Oberasbach (DE); SPROGAR, Christian, D-91088 Bubenreuth (DE); WEISS, Willy, D-90518 Altdorf (DE)
(74) Vertreter: Leissler-Gerstl, Gabriele
(86) Internationale Anmeldenummer: PCT/EP2000/002137
(87) Internationale Veröffentlichungsnummer: WO 2000/055264

(56) Entgegenhaltungen:
- EP-A- 0 861 657
- WO-A-95/11000
- CH-A- 195 333
- DE-A- 4 229 555
- DE-C- 19 643 356
- US-A- 4 741 774

## Beschreibung

Die Erfindung betrifft die Verwendung einer in organischen Lösungsmitteln löslichen Alkylcellulose und/oder Hydroxyethylcellulose zu Erhöhung der Zug-, Bruch- und Biegefestigkeit von Farbminen und solche Minen enthaltende Farbstifte.

Farbminen sind Pigmente, Farblacke oder Farbstoffe enthaltende Minen, die für Bunt-oder Farbstifte und Kosmetikstifte verwendet werden. Farbminen sollen einerseits leicht aufzutragen sein, müssen aber fest genug sein, um sie verarbeiten zu können. So sollen sie sich zum Beispiel bei Verwendung in Kosmetikstiften auf zarte Hautpartien sanft und weich auftragen lassen und dabei leicht und intensiv Farbe abgeben. Sie weisen deshalb im allgemeinen keine kristalline Struktur auf, sondern zeigen ein thixotropes Verhalten und haben erwünschterweise nur eine geringe Druckfestigkeit. Allerdings ist dadurch auch ihre Zug- und Biegefestigkeit gering, was sich nachteilig auswirkt. Beim Formen ist der Anteil an Ausschuß hoch, da die Minen leicht beim Entformen oder bei mechanischer Belastung brechen. Insbesondere bei Minen, die ein ungünstiges Verhältnis von Länge zu Durchmesser aufweisen, beispielsweise einen Durchmesser von 2 bis 6 mm bei einer Länge bis zu 25 bis 50 mm, ist die Gefahr des Abbrechens groß.

Besonders nachteilig erweist sich die geringe Festigkeit, insbesondere Zug- und Biegefestigkeit, wenn dünne Farbminen mit einem Durchmesser von weniger als 6 mm in einen Drehstift eingesetzt werden sollen, wo sie, eingesetzt in eine Drehmechanik, vor- und zurückgedreht werden können, aber nicht mechanisch gestützt werden.

Im Bereich der Kosmetikstifte sind beispielsweise sogenannte "Liner" vor allem für Lidstrich- und Lippenkonturenstifte, beliebt, die eingesetzt in Drehmechaniken angeboten werden. Diese Liner müssen nicht gespitzt werden und die Mine ist, wenn sie nicht verwendet wird, in der Linerhülse geschützt.

Farbminen werden üblicherweise hergestellt, indem eine homogenisierte Grundmasse auf Basis von Fett-Wachs-Mischungen, die außer den farbgebenden Pigmenten weitere Zusatzstoffe enthalten kann, in eine Form gegossen wird oder extrudiert wird. Für die Anwendung wird die Mine entweder in Holz eingebettet, in vorgeformte Hülsen aus Holz oder Kunststoff oder dergleichen eingegossen oder in eine Drehmechanik eingesetzt.

Wenn Gießformen verwendet werden, so treten in der ausgekühlten Form beachtliche Adhäsionskräfte auf, was trotz der Verwendung von Trennmitteln leicht zum Abreißen der Gießlinge und damit zu Ausschuß führt. Darüberhinaus sind umfangreiche Reinigungsarbeiten an der Gießanlage erforderlich. Auch das Einsetzen der Farbminen in eine Drehmechanik und das Herausdrehen der Mine führt häufig zu Bruch.

Es war daher eine Aufgabe der Erfindung, die bisher mit Farbminen verbundenen Nachteile zu beseitigen und bekannte Stiftmassen so zu verbessern, daß die Zug- und Biegefestigkeit erhöht wird, sodaß sogar dünne Minen gegossen und extrudiert werden können, ohne daß es beim Herausnehmen der Minen aus der Gießform oder beim Einsetzen der Mine in eine Drehmechanik zu einem Abbrechen oder einer Beschädigung der Minen kommt. Dabei sollen aber gleichzeitig die guten und erwünschten Eigenschaften, insbesondere die guten Applikationseigenschaften, solcher Minen nicht nachteilig verändert werden.

Die Lösung dieser Aufgabe findet sich in der Verwendung gemäß Anspruch 1. Es wird dazu zur Erhöhung der Zug-, Bruch- und Biegefestigkeit von Farbminen der Minenmasse 1 bis 50 Gew.-% in organischen Lösungsmitteln lösliche Alkylcellulose und/oder Hydroxyalkylcellulose, deren Alkylreste geradkettig oder verzweigt sein können und 2 bis 10 Kohlenstoffatome aufweisen, zugesetzt.

Überraschenderweise wurde festgestellt, daß der Zusatz einer in organischen Lösungsmitteln löslichen Alkylcellulose und/oder Hydroxyalkylcellulose, im folgenden auch als Cellulosederivat bezeichnet, der Minenmasse eine höhere Elastizität verleiht, ohne die guten Eigenschaften der Mine zu beeinträchtigen und ohne die Eigenschaften der anderen Inhaltsstoffe nachteilig zu verändern.

Es war zwar bereits bekannt, wasserlösliche Cellulosederivate als Bindemittel, insbesondere in der Schreibgeräteindustrie, zu verwenden. Der Zusatz solcher wasserlöslicher Cellulosederivate führt jedoch nicht zu einer Erhöhung der Zug- und Biegefestigkeit von Minen.

Weiterhin werden in EP-A 0 861 657 kosmetische Massen beschrieben, deren Filmbildungseigenschaften und Haftungseigenschaften durch Zusatz von Ethylcellulose verbessert werden sollen. Insbesondere sollen gelartige Massen hergestellt werden. Die der Erfindung zugrunde liegende Aufgabe löst diese Veröffentlichung jedoch nicht.

Die Grundmasse für Farbminen besteht üblicherweise aus Fett-, Wachs- und Ölrohstoffen. Dieser Grundmasse können zur Erzielung gewünschter Eigenschaften Zusatzstoffe zugesetzt werden. Der wichtigste Zusatzstoff sind Färbemittel, die in Form von Pigmenten, Farblacken oder Farbstoffen zugesetzt werden. Weiterhin werden zur Beeinflussung der Konsistenz Bindemittel und Füllstoffe verwendet. Gegebenenfalls werden zur Verbesserung der Haltbarkeit und Lagerfähigkeit Konservie-rungsmittel und Antioxidantien zugesetzt. Eine übliche Rezeptur für Farbminen enthält z.B. Wachse, wie Paraffin, Bienenwachs etc., Ölrohstoffe, wie hydriertes Pflanzenöl, Pigmente zum Anfärben, und ein bei Körpertemperatur flüchtiges Lösungsmittel, wie Cyclomethicone, das die Auftragbarkeit der Mine verbessert und nach dem Auftragen verdampft unter Zurücklassen eines haltbaren Farbstrichs. Die Grundmasse für die Farbmine kann, je nach Wunsch und Bedarf, weitere Zusatzstoffe enthalten, die dem Fachmann auf diesem Gebiet bekannt sind und hier keiner weiteren Erläuterung bedürfen. Falls Paraffin für die Grundmasse verwendet wird, sollte sein Anteil nicht zu hoch sein, da bei zu großen Mengen an Paraffin Unverträglichkeiten mit dem erfindungsgemäß eingesetzten Cellulosederivat auftreten können.

Einer derartigen üblichen Grundmasse für eine Farbmine wird erfindungsgemäß zur Erhöhung der Zug- und Biegefestigkeit ein Cellulosederivat, wie unten definiert, zugesetzt. Je nach Art der Grundmasse und des Verarbeitungsverfahrens kann der Anteil des Cellulosederivats variieren. In der Regel hat sich ein Anteil in einem Bereich von 1 bis 50 Gewichtsteilen, bezogen auf 100 Gewichtsteile der Grundmasse, als günstig erwiesen. Eine Menge von weniger als 1 Gewichtsteil hat keinen wesentlichen Einfluß mehr auf die Festigkeit, während bei einem Anteil von mehr als 50 Gewichtsteilen die Viskosität der Masse in einem für die Verarbeitung ungünstigen Bereich liegen kann.

Wenn die Masse unter hohem Druck extrudiert werden soll, kann der Anteil an Cellulosederivat im oberen Bereich liegen, während dann, wenn die Masse durch Gießen geformt werden soll, ein Anteil im unteren Bereich günstiger ist. Bevorzugt werden nicht mehr als 30 Gewichtsteile und besonders bevorzugt nicht mehr als 20 Gewichtsteile des Cellulosederivates zugegeben. Besonders günstige Ergebnisse wurden mit einem Zusatz von 3 bis 10 Gewichtsteilen Cellulosederivat pro 100 Gewichtsteilen Minengrundmasse erzielt.

Als Cellulosederivat wird erfindungsgemäß eine in organischen Lösungsmitteln lösliche Alkyl-cellulose und/oder Hydroxyalkylcellulose verwendet. Zur Lösung der Cellulosederivate können unter anderem als organische Lösungsmittel Fettaklohole, Fettsäuren und deren Ester verwendet werden. Als in organischen Lösungsmitteln lösliche Alkyl-cellulose und/oder Hydroxyalkylcellulosen werden insbesondere solche angesehen, von denen sich ein Teil in bis zu 100 Teilen organischem Lösungsmittel bei einer Temperatur von 100°C löst.

Besonders bevorzugt werden Cellulosederivate verwendet, die in Fettalkoholen oder, Fettsäureestern löslich sind, insbesondere solche, von denen 1 g in 1 bis 100 g des Fettalkohols, oder Esters bei 100°C gelöst wird.

Der Alkylrest der Alkylcellulosen und/oder Hydroxyalkylcellulosen kann geradkettig oder verzweigt sein und weist 2 bis 10 Kohlenstoffatome auf. Bevorzugt werden Cellulosederivate verwendet, deren Alkylanteil 2 bis 6, insbesondere 2 oder 3 Kohlenstoffatome aufweist, da diese leicht verfügbar sind. Besonders bevorzugt wird Ethylcellulose ggf. gemischt mit anderen Cellulosederivaten eingesetzt.

Die Löslichkeit der Cellulosederivate hängt unter anderem von ihrem Substitutionsgrad ab. Bevorzugt werden daher solche Cellulosederivate inbetracht gezogen, deren Substitutionsgrad höher als 1,4 ist. Besonders bevorzugt wird Ethylcellulose mit einem Substitutionsgrad von 2,1 bis 2,6 oder eine Mischung verschiedener Derivate mit einem Substitutionsgrad in diesem Bereich verwendet.

Mit dem erfindungsgemäßen Verfahren gelingt es, eine Farbmine bereitzustellen, deren Zug- und Biegefestigkeit verbessert sind und die auch eine verbesserte Elastizität aufweist. Sie ist stabil genug, um in einer Drehmechanik herausgedreht zu werden ohne abzubrechen und hält freitragend. Die Auftragseigenschaften sind gut, sie kann beispielsweise in angenehmer Weise auf Haut aufgetragen werden.

Aufgrund dieser Verbesserung der mechanischen Eigenschaften können Minen hergestellt werden, bei denen das Verhältnis von Länge zu Durchmesser größer als 5:1 oder 8:1 und sogar 10:1 und mehr sein kann.

Die Farbmine wird in an sich bekannter Weise hergestellt, indem die Komponenten der Grundmasse und das erfindungsgemäß verwendete Cellulosederivat vermischt und homogenisiert werden und die entstehende Masse dann, in der Regel durch Gießen oder Extrudieren zu einer Mine geformt wird. Üblicherweise wird zur Herstellung der Minen die Masse entweder in Formen gegossen und nach dem Abkühlen entformt oder in die Halteteile einer Drehmechanik eingesetzt oder durch ein Halteteil direkt in eine entsprechende Form eingegossen und nach dem Abkühlen in die Drehmechanik zurückgedreht oder aber in ein entsprechendes Teil einer Drehmechanik eingegossen und dort erstarren gelassen. Bevorzugt wird die erfindungsgemäße Farbmine mit einem Gießverfahren hergestellt.

Es wurde allerdings gefunden, daß in organischen Lösungsmitteln lösliche Cellulosederivate sich nicht immer problemlos mit der Grundmasse für eine Farbmine vermischen lassen.

Bevorzugt wird daher ein Verfahren zur Herstellung von Farbminen verwendet, bei dem man eine in organischen Lösungsmitteln lösliche Alkylcellulose und/oder Hydroxyalkylcellulose in einem kosmetisch annehmbaren Lösungmittel löst, die Lösung mit der Stiftmasse vermischt und in an sich bekannter Weise zu einer Mine weiterverarbeitet.

Das erfindungsgemäß eingesetzte Cellulosederivat wird bevorzugt in einem für das Gebiet der Kosmetik üblichen Lösungsmittel gelöst. Bevorzugt wird ein linearer oder verzweigter Fettalkohol mit einer Kettenlänge von 7 bis 50 Kohlenstoffatomen, besonders bevorzugt mit 12 bis 34 Kohlenstoffatomen und insbesondere 16 bis 24 Kohlenstoffatomen, eine lineare oder verzweigte, gesättigte oder ungesättigte Fettsäure, die bevorzugt eine Kettenlänge von 12 bis 24 Kohlenstoffatomen aufweist, wobei die längerkettigen Fettsäuren insbesondere bei erhöhter Temperatur eingesetzt werden, oder ein Ester einer Fettsäure mit einem kürzerkettigen Alkohol, insbesondere Isopropylmyristat, Isopropylpalmitat oder Myristinsäure ggf. in Gemisch mit Fettalkoholen verwendet. Es können auch Gemische aus den obengenannten Alkoholen und/oder Fettsäuren und/oder Estern verwendet werden. Als besonders geeignet haben sich Cetylalkohol, Stearylalkohol, Isostearylalkohol und Behenylalkohol, sowie deren Gemische erwiesen.

Das Cellulosederivat wird in dem Lösungsmittel gelöst, gegebenenfalls bei erhöhter Temperatur, und dann der Grundmasse zugegeben. Da die Grundmasse häufig bei erhöhter Temperatur homogenisiert wird, ist es bevorzugt, die das Cellulosederivat enthaltende Lösung vor dem Vermischen ebenfalls auf diese erhöhte Temperatur zu bringen.

Die Grundmasse wird mit dem Cellulosederivat vermischt und dann in an sich bekannter Weise weiter verarbeitet, z.B. durch Extrudieren oder Gießen, bevorzugt durch Gießen.

In einer bevorzugten Ausführungsform wird das Cellulosederivat in einem kosmetisch annehmbaren Lösungsmittel, bevorzugt einem Fettalkohol, einer Fettsäure oder einem Ester einer Fettsäure und eines Fettalkohols oder einer Mischung davon, gelöst, die Wachs- und Fettkomponenten werden aufgeschmolzen, beides wird miteinander vermischt und homogenisiert, dann werden als Färbemittel Pigmente, Farblacke und/oder Farbstoffe und gegebenenfalls Hilfsstoffe zugegeben und die Grundmasse dann in eine Form eingegossen und nach dem Abkühlen entformt.

Erfindungsgemäß werden Farbminen erhalten, die problemlos verarbeitet werden können aufgrund ihrer gegenüber bisher bekannten Minen verbesserten Festigkeitseigenschaften. Da sie weder bei der Herstellung noch bei der Anwendung abbrechen, können sie ohne Probleme zu Stiften verarbeitet werden.

Gegenstand der Erfindung ist daher auch ein Farbstift, der eine Mine und eine Hülse umfaßt, wobei die Mine aus einer üblichen Farbminenmasse besteht, der 1 bis 50 Gew.-% von in organischen Lösungsmitteln löslicher Alkyl cellulose und/oder Hydroxyalkylcellulose zugesetzt wurden. Die Hülse besteht aus natürlichen oder synthetischen Materialien.

Die mit dem erfindungsgemäßen Verfahren erhaltenen Farbminen sind so stabil, dass sie durch Extrudieren und Gießen verarbeitet werden können und außerdem haben sie eine so hohe Biege- und Zugfestigkeit, daß sie in eine Drehmechanik eingesetzt werden können und ohne abzubrechen heraus- und hereingedreht werden können. Ebenso sind sie geeignet, um in Hülsenrohlinge eingelegt zu werden und zu Stiften verarbeitet zu werden.

Bevorzugt wird die erfindungsgemäß erhaltene Farbmine für Farbstifte und Kosmetikstifte, besonders bevorzugt für Kosmetikstifte eingesetzt. Aufgrund ihrer vorteilhaften Eigenschaften kann die erfindungsgemäß erhaltene Farbmine zur Herstellung von Lidstrich-, Khol-, Augenbrauen- und Lippenkonturstiften verwendet werden.

Wegen ihrer erhöhten Zug- und Biegefestigkeit sind die erfindungsgemäß erhaltenen Minen zur Verwendung in kosmetischen "Linern", die eine Drehmechanik aufweisen, besonders gut geeignet. In solchen Stiften haben die Minen einen Durchmesser von maximal 6 mm, bei einer Länge von bis zu 80 mm. Daher werden an die mechanische Festigkeit solcher Stifte sehr hohe Anforderungen gestellt, die jedoch von den erfindungsgemäß erhaltenen Farbminen erfüllt werden.

Die Erfindung wird mit den folgenden Beispielen erläutert.

### Beispiel 1

### Herstellung eines Eyeliner-Stiftes

Es wurde eine erfindungsgemäße Farbmine mit der in der Tabelle 1 angegebenen Rezeptur hergestellt. Zum Vergleich wurde eine Mine aus der identischen Grundmasse hergestellt, der jedoch das erfindungsgemäß wesentliche Cellulosederivat fehlte. Die Rezeptur für beide Massen ist der folgenden Tabelle 1 zu entnehmen, wobei alle Mengenangaben in Gew.-% angegeben sind:

**Tabelle 1**

| **INCI-Name** | **Beispiel 1** | **Vergleichsbeispiel 1** |
|---|---|---|
| Colorants | 33,300 | 33,300 |
| Ethylcellulose | 1,500 | - |
| Isostearyl Alcohol | 5,900 | 5,900 |
| Stearyl Alcohol | 5,900 | 5,900 |
| Hydrogenated Vegetable Oil | 6,700 | 6,700 |
| Paraffin | 6,700 | 6,700 |
| Cyclomethicone | 40,000 | 41,500 |
| **Summe** | **100,000** | **100,000** |

Für die Mine von Beispiel 1 wurde die Ethylcellulose zu der der Mischung aus Isostearyl Alcohol und Stearyl Alcohol zugegeben. Die Mischung wurde dann unter Rühren auf 65 bis 90°C erwärmt und auf dieser Temperatur gehalten, bis alles gelöst war. Getrennt wurden Hydrogenated Vegetable Oil und Paraffin aufgeschmolzen und dann zu der Ethylcelluloselösung zugegeben. Anschließend wurden die Pigmente zugegeben und dann die gesamte Mischung in üblicher Weise homogenisiert. Nach Zusatz von Cyclomethicone wurde die Masse in bekannter Weise in eine Form eingegossen und nach dem Abkühlen und Festwerden entformt. Man erhielt einen Eyelinerstift mit guter Abgabe, bei weichem Auftrag und guter Stabilität.

Zum Vergleich wurden die Bestandteile der Rezeptur von Vergleichsbeispiel 1 in equivalenter Weise vermischt, indem Hydrogenated Vegetable Oil und Paraffin aufgeschmolzen wurden und anschließend die Pigmente und Isostearyl Alcohol und Stearyl Alcohol zugegeben wurden. Zum Schluß wurde das Cyclomethicone der Masse zugesetzt, und die Masse in eine Form eingegossen. Es wurde eine Masse mit vergleichbarer Abgabe erhalten, die jedoch eine ungenügende Bruchfestigkeit aufwies. Diese Masse ließ sich deshalb nur schwer und mit erheblichem Ausschuß aus den Formen entnehmen und in der Drehmechanik zurückdrehen. Beim Auftragen brachen die Minen mit einem Durchmesser von 2,5 mm sehr leicht ab.

### Beispiel 2

### Herstellung eines Lippenkonturenstiftes

Es wurde eine Masse für eine Farbmine hergestellt, die für einen Lippenkonturenstift geeignet war. Zum Vergleich wurde eine Mine für einen Lippenkonturenstift hergestellt, deren Grundmasse identisch war mit der von Beispiel 2, der jedoch die erfindungsgemäß wesentliche Ethylcellulose fehlte. Die Rezepturen für beide Mischungen sind in der folgenden Tabelle 2 jeweils in Gew.-% angegeben.

**Tabelle 2**

| **INCI-Name** | **Beispiel 2** | **Vergleichsbeispiel 2** |
|---|---|---|
| Colorants | 21,300 | 21,300 |
| Ethylcellulose | 3,150 | - |
| Isostearyl Alcohol | 5,250 | 5,250 |
| Cetylpalmitat | 5,250 | 5,250 |
| Beeswax | 23,600 | 23,600 |
| Synthetic Wax | 3,150 | 3,150 |
| PPG-12/SMDI Copolymer | 1,600 | 1,600 |
| Cyclomethicone | 36,700 | 39,850 |
| **Summe** | **100,000** | **100,000** |

Für die Mine von Beispiel 2 wurde die Ethylcellulose zu einer erwärmten Mischung aus Isostearyl Alcohol und Cetylpalmitat zugegeben und dann unter Rühren auf 65 bis 90°C erwärmt und bei dieser Temperatur gehalten, bis alles gelöst war. Getrennt davon wurden Beeswax, Synthetic Wax und PPG-12/SMDI Copolymer aufgeschmolzen und dann zu der Ethylcelluloselösung zugegeben. Anschließend wurden die Pigmente zugegeben und dann die gesamte Mischung in üblicher Weise homogenisiert. Nach dem Zusatz von Cyclomethicone wurde die Masse in bekannter Weise in eine Form eingegossen und nach dem Abkühlen und Festwerden entformt. Es wurde ein Lippenkonturenstift mit guter Abgabe, weichem Auftrag und guter Stabilität erhalten.

Zum Vergleich wurde in äquivalenter Weise die Masse gemäß der Rezeptur von Vergleichsbeispiel 2 verarbeitet. Dazu wurden Beeswax, Synthetic Wax und PPG-12/SMDI Copolymer aufgeschmolzen und dann Pigmente, Isostearyl Alcohol, Cetylpalmitat zugegeben und schließlich Cyclomethicone zugesetzt. Die Masse wurde dann ebenfalls in bekannter Weise in eine Form eingegossen und nach dem Abkühlen und Festwerden entformt. Die mit der Masse von Vergleichsbeispiel 2 erhaltene Mine wies eine vergleichbare Abgabe auf, hatte jedoch eine ungenügende Druckfestigkeit. Sie war nur schwer und mit erheblichem Ausschuß aus den Formen zu entnehmen und in die Drehmechanik zurückzudrehen. Beim Auftragen brach die Mine mit einem Druchmesser von 2,5 mm sehr leicht ab.

Die Beispiele zeigen, daß durch den erfindungsgemäßen Zusatz eines Cellulosederivats zu einer üblichen Grundmasse für Farbminen die mechanischen Eigenschaften, insbesondere die Zug- und Biegefestigkeit sowie die Bruchfestigkeit stark verbessert werden, ohne die vorteilhaften Auftragseigenschaften in negativer Weise zu beeinflussen. Die Minen der vorliegenden Erfindung sind daher besonders geeignet für Stifte mit einer Drehmechanik, wo die Mine einer höheren mechanischen Belastung ausgesetzt ist, als bei spitzbaren, holz- und kunststoffgefaßten Stiften. Natürlich sind die erfindungs-gemäßen Farbminen auch für holz- oder kunststoffgefaßte Stifte geeignet.

### Beispiel 3

Es wurden Minenmassen hergestellt aus Japanwachs, Isostearyl Alcohol und Ethylcellulose, ohne Pigmente, um die mechanischen Eigenschaften von daraus hergestellten Minen zu prüfen. Der Schwachpunkt bei dünn gegossenen Minen, die in eine Drehmechanik eingesetzt werden, ist die Übergangsstelle unmittelbar am Minenhalter, wo die Minen bevorzugt abbrechen, es wurden Massen hergestellt mit dem folgenden angegebenen Zusammensetzungen. Aus dieser Masse wurden Minen mit einer Stärke mit 3 mm und einer Länge von 34 mm gegossen und dann verschiedenen Tests unterzogen.

**Tabelle**

| | 1 | 2 | 3 |
|---|---|---|---|
| Japanwachs (Gew.-teile) | 50.000 | 50.000 | 50.000 |
| Isostearyl Alcohol (Gew.-teile) | 25.000 | 25.000 | 25.000 |
| Ethylcellulose (Gew.-teile) | - | 2.500 | 5.000 |

### 3.a) Formtest

Die erhaltene Masse wurde in Hülsenrohlinge gegossen und dann wurde versucht, den Formling zu entformen. Nur die Minen mit der Rezeptur 3 ließen sich ohne Probleme und ohne Hilfsmittel, wie Trennmittel, Druckluft ect. entformen. Bei den Minen mit der Rezeptur 2 entstand erheblicher Ausschuß. Mit der Masse der Rezeptur 1 konnten keine Minen entformt werden.

### 3.b) Gießtest

Weiterhin wurden die Massen mit den Rezepturen 1, 2 und 3 in einer Drehmechanik getestet. Dazu wurde eine teilmontierte Mechanik auf eine Metallform aufgesetzt und die jeweilige Masse dann durch das Halteteil hindurch in die Form gegossen. Folgendes Ergebnis wurde erhalten:

| | | |
|---|---|---|
| Rezeptur | 1. | 20 von 20 Abreißer |
| Rezeptur | 2. | 4 von 20 Abreißer |
| Rezeptur | 3. | 0 von 20 Abreißer |

Dies zeigt, daß die erfindungsgemäß erhaltene Minenmasse eine hohe Stabilität aufweist und ohne Probleme gegossen und entformt werden kann.

### 3.c) Falltest

Mit den aus den 3 Rezepturen gegossenen Minen wurde ein Falltest durchgeführt. Hierzu ließ man die komplette Mechanik mit eingesetzter Mine, in einem Führungsrohr 3 mal aus 30 cm Höhe mit der Spitze voran auf ein harte Unterlage fallen. Das Ergebnis des Falltests war wie folgt:

| | | |
|---|---|---|
| Rezeptur | 1. | 20 von 20 Abreißer |
| Rezeptur | 2. | 9 von 20 Abreißer |
| Rezeptur | 3. | 0 von 20 Abreißer |

Dieser Test zeigt, daß die erfindungsgemäß erhaltene Farbmine die eine ausgezeichnete Festigkeit aufweist.

### 3.d) Zugtest

Mit Minen, die aus den 3 Rezepturen hergestellt wurden, wurde ein Zugtest durchgeführt. Dazu wurde auf eine in eine Drehmechanik eingesetzte Mine in axialer Richtung eine Zugkraft ausgeübt und der Wert festgestellt, bei dem die Mine am Halteteil abriß. Folgende Ergebnisse wurden erhalten:

| | | |
|---|---|---|
| Rezeptur | 1. | 0,001 N bis 0,005 N |
| Rezeptur | 2. | 0,020 N bis 0,040 N |
| Rezeptur | 3. | 2,040 N bis 3,400 N |

Dieser Test zeigt, daß die erfindungsgemäß erhaltene Farbmine eine ausgezeichnete Zugfestigkeit aufweist.

### 3.e) Biegetest

Mit aus den 3 Rezepturen erhaltenen Minen wurde ein Biegetest durchgeführt. Dazu wurde die Mine jeweils vollständig aus der Drehmechanik heraus gedreht und dann, 34 mm vom Halteteil entfernt, auf die Mine ein Kraft einwirken gelassen. Die Auslenkung der Minenspitze, bevor diese abbricht, wird gemessen. Folgende Ergebnisse wurden erhalten:

| | | |
|---|---|---|
| Rezeptur | 1. | Nicht messbar |
| Rezeptur | 2. | < als 1 mm |
| Rezeptur | 3. | 3 mm bis 6 mm |

Dieser Test zeigt, daß die erfindungsgemäß erhaltenen Farbminen eine ausgezeichnete Biegefestigkeit aufweisen.

Die durchgeführten Tests beweisen, daß es erfindungsgemäß möglich ist, selbst bei sehr dünnen Minen die Zug- und Biegefestigkeit erheblich zu steigern, so daß diese Minen in Drehmechaniken ohne Probleme verwendet werden können.

## Patentansprüche

1. Verwendung einer in organischen Lösungsmitteln löslichen Alkyl cellulose und/oder Hydroxyalkylcellulose, deren Alkylreste geradkettig oder verzweigt sein können und 2 bis 10 Kohlenstoffatome aufweisen zur Erhöhung der Zug-, Bruch- und Biegefestigkeit von Farbminen, wobei der Minenmasse 1 bis 50 Gew.-% der Cellulose zugesetzt werden.

2. Verwendung nach Anspruch 1,
**dadurch gekennzeichnet, dass** 1 bis 30 Gew.-% von in organischen Lösungsmittel löslicher Alkyl cellulose und/oder Hydroxyalkylcellulose zugesetzt werden.

3. Verwendung nach Anspruch 1 oder Anspruch 2,
**dadurch gekennzeichnet, dass** 3 bis 10 Gew.-% von in organischen Lösungsmitteln löslicher Alkyl cellulose und/oder Hydroxyalkylcellulose zugesetzt werden.

4. Verwendung nach einem der vorhergehenden Ansprüche,
**dadurch gekennzeichnet, dass** eine in organischen Lösungsmitteln lösliche Ethylcellulose zugesetzt wird.

5. Verwendung nach einem der vorhergehenden Ansprüche,
**dadurch gekennzeichnet, dass** eine Alkyl cellulose und/oder Hydroxyalkylcellulose zugesetzt wird, von der sich ein Teil in 1 bis 100 Teilen eines Fettalkohols, einer Fettsäure oder eines Esters einer Fettsäure mit einem Fettalkohol bei 100°C löst.

6. Verwendung nach einem der vorhergehenden Ansprüche,
**dadurch gekennzeichnet, dass** zur Lösung der Alkylcellulose und/oder Hydroxyalkylcellulose ein linearer oder verzweigter Fettalkohol mit einer Kettenlänge von 7 bis 50 Kohlenstoffatomen, bevorzugt 12 bis 34 Kohlenstoffatomen oder ein Ester einer linearen oder verzweigten, gesättigten oder ungesättigten Fettsäure mit einer Kettenlänge von 12 bis 24 Kohlenstoffatomen verwendet wird.

7. Verwendung nach einem der vorhergehenden Ansprüche,
**dadurch gekennzeichnet, dass** zur Lösung des Cellulosederivats Isopropylmyristat, Isopropylpalmitat, Myristinsäure, Cetylalkohol, Stearylalkohol, Isostearylalkohol, Behenylalkohol oder ein Gemisch davon verwendet wird.

8. Verwendung nach einem der vorhergehenden Anspüche,
**dadurch gekennzeichnet, dass** man aus der Minenmasse Farbminen formt, deren Verhältnis von Länge zu Durchmesser mindestens 5:1, bevorzugt mindestens 8:1 ist.

9. Verwendung nach einem der vorhergehenden Ansprüche,
**dadurch gekennzeichnet, dass** man aus der Minenmasse Kosmetikminen formt.

10. Verwendung nach einem der vorhergehenden Ansprüche,
**dadurch gekennzeichnet, dass** man die Minen durch Gießen formt.

11. Farbstift umfassend eine Farbmine und eine Hülse, wobei die Mine neben den üblichen Inhaltsstoffen 1 bis 50 Gew.-%, bezogen auf das Gewicht der Mine, von in organischen Lösungsmittel löslicher Alkyl-cellulose und/oder Hydroxyalkylcellulose, deren Alkylreste geradkettig oder verzweigt sein können und 2 bis 10 Kohlenstoffatome aufweisen, enthält, wobei die Mine so ausgebildet ist, dass das Verhältnis von Länge zu Durchmesser mindestens 5:1 beträgt.

12. Farbstift nach Anspruch 11,
**dadurch gekennzeichnet, dass** die Farbmine einen Durchmesser von 1 bis 6 mm hat.

13. Farbstift nach Anspruch 11 oder 12,
**dadurch gekennzeichnet, dass** er eine Hülse umfasst, die mit einer Drehmechanik ausgerüstet ist, in die eine Farbmine eingesetzt ist.

14. Farbstift nach Anspruch 13,
**dadurch gekennzeichnet, dass** die Mine frei tragend ist.

15. Farbstift nach einem des Ansprüche 11 bis 14 umfassend eine Farbmine, deren Durchmesser kleiner oder gleich 6 mm ist und deren Länge 25 bis 80 mm beträgt, eingesetzt in die Drehmechanik eines Drehstiftes.

16. Farbstift nach einem der Ansprüche 11 bis 15,
**dadurch gekennzeichnet, dass** es ein Kosmetikstift ist.

17. Farbstift nach Anspruch 16,
**dadurch gekennzeichnet, dass** es ein Augenbrauen-, Khol-, Lidschatten-oder Lippenkonturenstift ist.

## Claims

1. Use of an alkylcellulose and/or hydroxyalkylcellulose soluble in organic solvents, the alkyl radicals of which may be straight-chain or branched and which have 2 to 10 carbon atoms, to increase the tensile strength, breaking strength and flexural strength of coloured leads, wherein 1 to 50 wt.% of the cellulose is added to the lead mass.

2. Use according to claim 1,
**characterised in that** 1 to 30 wt.% of alkylcellulose and/or hydroxyalkylcellulose soluble in organic solvents is added.

3. Use according to claim 1 or 2,
**characterised in that** 3 to 10 wt.% of alkylcellulose and/or hydroxyalkylcellulose soluble in organic solvents is added.

4. Use according to one of the preceding claims,
**characterised in that** an ethylcellulose soluble in organic solvents is added.

5. Use according to one of the preceding claims,
**characterised in that** an alkylcellulose or hydroxyalkylcellulose is added, one part of which dissolves in 1 to 100 parts of a fatty alcohol, a fatty acid or an ester of a fatty acid with a fatty alcohol at 100°C.

6. Use according to one of the preceding claims,
**characterised in that** a linear or branched fatty alcohol with a chain length of 7 to 50 carbon atoms, preferably 12 to 34 carbon atoms, or an ester of a linear or branched, saturated or unsaturated fatty acid with a chain length of 12 to 24 carbon atoms, is used to dissolve the alkylcellulose and/or hydroxyalkylcellulose.

7. Use according to one of the preceding claims,
**characterised in that** isopropyl myristate, isopropyl palmitate, myristic acid, cetyl alcohol, stearyl alcohol, isostearyl alcohol, behenyl alcohol or a mixture thereof is used to dissolve the cellulose derivative.

8. Use according to one of the preceding claims,
**characterised in that** coloured leads are formed from the lead mass, the length to diameter ratio of which is at least 5:1, preferably at least 8:1.

9. Use according to one of the preceding claims,
**characterised in that** cosmetic leads are formed from the lead mass.

10. Use according to one of the preceding claims,
**characterised in that** the leads are formed by casting.

11. A coloured pencil comprising a coloured lead and a casing, wherein, in addition to the conventional constituents, the lead contains 1 to 50 wt.%, based on the weight of the lead, of alkylcellulose and/or hydroxyalkylcellulose soluble in organic solvents, the alkyl radicals of which may be straight-chain or branched and which have 2 to 10 carbon atoms, the lead being designed such that its length to diameter ratio is at least 5:1.

12. The coloured pencil according to claim 11,
**characterised in that** the coloured lead has a diameter of 1 to 6 mm.

13. The coloured pencil according to claim 11 or 12,
**characterised in that** it comprises a casing, which is equipped with a turning mechanism into which a coloured lead is inserted.

14. The coloured pencil according to claim 13,
**characterised in that** the lead is self-supporting.

15. The coloured pencil according to one of claims 11 to 14, comprising a coloured lead the diameter of which is less than or equal to 6 mm and the length of which is 25 to 80 mm, inserted into the turning mechanism of a rotary pencil.

16. The coloured pencil according to one of claims 11 to 15,
**characterised in that** it is a cosmetic pencil.

17. The coloured pencil according to claim 16,
**characterised in that** it is an eyebrow pencil, kohl pencil, eyeshadow pencil or lipliner pencil.

## Revendications

1. Utilisation d'une alkylcellulose et/ou d'une hydroxyalkylcellulose soluble dans des solvants organiques, dont les radicaux alkyle peuvent être linéaires ou ramifiés et présentent 2 à 10 atomes de carbone, pour augmenter la résistance à la traction, à la rupture et à la flexion de mines de crayon de couleur, dans laquelle on ajoute à la composition ou masse de mine 1 à 50 % en poids de cellulose.

2. Utilisation selon la revendication 1 ou la revendication 2, **caractérisée en ce que** l'on ajoute 1 à 30 % en poids d'alkylcellulose et/ou d'hydroxyalkylcellulose soluble dans des solvants organiques.

3. Utilisation selon la revendication 1, **caractérisée en ce que** l'on ajoute 3 à 10 % en poids d'alkylcellulose et/ou d'hydroxyalkylcellulose soluble dans des solvants organiques.

4. Utilisation selon l'une des revendications précédentes, **caractérisée en ce que** l'on ajoute une éthylcellulose soluble dans des solvants organiques.

5. Utilisation selon l'une des revendications précédentes, **caractérisée en ce que** l'on ajoute une alkylcellulose et/ou une hydroxyalkylcellulose dont une partie se dissout dans 1 à 100 parties d'un alcool gras, d'un acide gras ou d'un ester d'un acide gras avec un alcool gras à 100 °C.

6. Utilisation selon l'une des revendications précédentes, **caractérisée en ce que** pour dissoudre l'alkylcellulose et/ou l'hydroxyalkylcellulose, on utilise un alcool gras linéaire ou ramifié présentant une longueur de chaîne comprise entre 7 et 50 atomes de carbone, de préférence entre 12 et 34 atomes de carbone, ou un ester d'un acide gras linéaire ou ramifié, saturé ou insaturé, présentant une longueur de chaîne comprise entre 12 et 24 atomes de carbone.

7. Utilisation selon l'une des revendications précédentes, **caractérisée en ce que** pour dissoudre le dérivé de cellulose, on utilise du myristate d'isopropyle, du palmitate d'isopropyle, de l'acide myristique, de l'alcool de cétyle, de l'alcool de stéaryle, de l'alcool d'isostéaryle, de l'alcool de béhényle ou un mélange de ceux-ci.

8. Utilisation selon l'une des revendications précédentes, **caractérisée en ce que** l'on forme à partir de la composition ou masse de mine des mines de crayon de couleur dont le rapport entre longueur et diamètre est d'au moins 5:1, de préférence d'au moins 8:1.

9. Utilisation selon l'une des revendications précédentes, **caractérisée en ce que** l'on forme des mines cosmétiques à partir de la composition ou masse de mine.

10. Utilisation selon l'une des revendications précédentes, **caractérisée en ce que** l'on forme des mines par coulée.

11. Crayon de couleur comportant une mine et une douille, dans lequel la mine, en plus des ingrédients usuels, comprend 1 à 50 % en poids, sur la base du poids de la mine, d'alkylcellulose et/ou d'hydroxyalkylcellulose soluble dans des solvants organiques, dont les radicaux alkyle peuvent être linéaires ou ramifiés et présentent 2 à 10 atomes de carbone, la mine étant réalisée de façon telle que le rapport entre longueur et diamètre est d'au moins 5:1.

12. Crayon de couleur selon la revendication 11, **caractérisé en ce que** la mine de crayon a un diamètre compris entre 1 et 6 mm.

13. Crayon de couleur selon la revendication 11 ou 12, **caractérisé en ce qu'**il comprend une douille qui est équipée d'un mécanisme à rotation dans lequel est utilisée la mine de couleur.

14. Crayon de couleur selon la revendication 13, **caractérisé en ce que** la mine est à portée libre.

15. Crayon de couleur selon l'une des revendications 11 à 14, comportant une mine de couleur, dont le diamètre est inférieur ou égal à 6 mm et dont la longueur est comprise entre 25 et 80 mm, utilisée dans le mécanisme à rotation d'un crayon rotatif.

16. Crayon de couleur selon l'une des revendications 11 à 15, **caractérisé en ce qu'**il s'agit d'un crayon cosmétique.

17. Crayon de couleur selon la revendication 16, **caractérisé en ce qu'**il s'agit d'un crayon à sourcils, d'un crayon à kôhl, d'un crayon de fard à paupières ou d'un crayon pour contour des lèvres.
